# EUROPEAN PATENT APPLICATION

(11) **EP 4 316 426 A1**
(43) Date of publication of application: **07.02.2024**
(21) Application number: 21946694.3
(22) Date of filing: 25.08.2021
(51) Int. Cl.: A61F 2/24

(54) **ARTIFICIAL HEART VALVE AND DELIVERY SYSTEM THEREFOR**

(30) Priority: 25.06.2021 CN 202110709244; 25.06.2021 CN 202121424835 U
(71) Applicant: Shanghai Trulive Medtech Co., Ltd, Shanghai 201206 (CN); Jiangsu Trulive Medtech Co., Ltd, Rudong County Nantong Jiangsu 226499 (CN)
(72) Inventor: ZHANG, Haijun, Shanghai 201206 (CN); HE, Dong, Shanghai 201206 (CN); LIU, Xiang, Shanghai 201206 (CN); WEN, Jing, Shanghai 201206 (CN)
(74) Representative: Nordmeyer, Philipp Werner
(86) International application number: PCT/CN2021/114546
(87) International publication number: WO 2022/267210

(57) **Abstract**

An artificial heart valve and a delivery system therefor are disclosed. The artificial heart valve includes an outer stent (1) and an inner stent (2), the outer stent (1) is detachably connected to the inner stent (2), or when the artificial heart valve is in use, the inner stent (2) is located inside the outer stent (1), the inner stent (2) is not connected to the outer stent (1), the outer stent (1) includes an outer stent frame (11) and one-time leaflets (12) provided on the outer stent frame (11), the inner stent (2) includes an inner stent frame (21) and prosthetic leaflets (22) disposed within the inner stent frame (21). The delivery system includes a delivery sheath (31) and, disposed within the delivery sheath (31), a guidewire lumen (32) and a stent catheter (33), the stent catheter (33) is configured to be suitable for engagement with the artificial heart valve. According to the artificial heart valve and the delivery system therefor, the delivery profile of the delivery system is reduced, and the safety of implantation and the universality of the profile are improved.

## Description

### TECHNICAL FIELD

The present invention relates to the field of medical device and, in particular, to an artificial heart valve and a delivery system therefor.

### BACKGROUND

Heart valves are openable and closeable flap-like structures in the organs of humans and some animals. The human heart has four valves: the aortic valve connecting the left ventricle to the aorta; the pulmonary valve connecting the right ventricle to the pulmonary artery; the mitral valve connecting the left atrium to the left ventricle; and the tricuspid valve connecting the right atrium to the right ventricle. All these valves function like one-way valves which allow blood flow in the forward direction but prevent its backward flow.

With the development of social economy and the aging of population, the incidence of valvular heart disease has increased significantly. Studies have shown that this figure has reached up to 13.3% among those 75 years or older. At present, traditional surgical treatment remains the first choice for patients with severe valvular disease. However, for those with advanced ages, complications in multiple organs, a history of thoracotomy or poor heart functions, the traditional surgical operation is associated with high risk and high mortality or even precludes some patients. The tricuspid valve, also known as the right atrioventricular valve, has a similar structure to the mitral valve - it similarly includes the leaflets, annulus, chordae tendineae, papillary muscles and cardiac muscles. Transcatheter tricuspid valve replacement/repair has attracted extensive attention amongst academics and researchers thanks to its advantages such as no need for thoracotomy, minor trauma and rapid recovery.

Existing tricuspid valve designs includes either a double-layer or single-layer stent. A single-layer stent is often used with a valve having a relatively large radius. However, compared with a radially smaller valve, a radially grater valve would be subject to higher blood pulsation pressure over cardiac cycles, which would more seriously affect the stent's fatigue strength. Moreover, it is difficult for a single-layer stent to maintain its own shape or that of a valve that it supports over cardiac cycles. In contrast, a double-layer stent can be used with a valve having a smaller diameter and can distribute the functions of, for example, attaching prosthetic leaflets, anchoring and sealing, over different constituent layers. Thus, it allows the implant to perform its intended therapeutic function while not affecting the normal functioning of the rest of the heart. However, double-layer stents typically suffer from the problem of a very high overall delivery profile, which may lead to high delivery difficulties, a narrower scope of application and potential safety hazards during use.

### SUMMARY OF THE INVENTION

In view of the above-discussed shortcomings of the prior art, it is an objective of the present invention to provide an artificial heart valve and a delivery system thereof, which can overcome the problems with the prior art.

In order to achieve the above and other objectives, the present invention provides an artificial heart valve including an outer stent and an inner stent. The inner stent is detachably coupled to the outer stent, or is seated inside the outer stent while not being coupled thereto during use of the artificial heart valve. The outer stent includes an outer stent frame and one-time leaflets provided on the outer stent frame. The inner stent includes an inner stent frame and prosthetic leaflets disposed within the inner stent frame.

The present invention also provides a delivery system for an artificial heart valve. The delivery system includes a delivery sheath and, disposed within the delivery sheath, a guidewire lumen and a stent catheter. The stent catheter is configured to be suitable for engagement with the artificial heart valve.

The present invention also provides a heart valve replacement system including the artificial heart valve and the delivery system.

The artificial heart valve and the delivery system of the present invention have the benefits as follows:
1. The stent assembly can be loaded in the delivery system without radially overlapping of the stents as in conventional double-layer stents, thus allowing the delivery system to have a much lower profile.
2. The lower profile means reduced delivery risks, higher implantation safety and a wider scope of application of the delivery system.
3. The lower profile enables the valve and system to be used on more patients such as those with smaller femoral veins.
4. When crimped into the delivery system, the stent assembly is less stiff than conventional double-layer stents and can thus pass through a tortuous vessel segment such as the arch of the aorta.

### BRIEF DESCRIPTION OF THE DRAWINGS

Fig. 1 shows a schematic cross-sectional view of an artificial heart valve according to the present invention.
Fig. 2 shows a schematic cross-sectional view of an outer stent in an artificial heart valve according to the present invention, in which a skirt is shown in hatch.
Fig. 3 shows a schematic cross-sectional view of an inner stent in an artificial heart valve according to the present invention.
Fig. 4-1 shows a schematic cross-sectional view of a delivery system for an artificial heart valve according to the present invention.
Fig. 4-2 shows a schematic cross-sectional view of another delivery system for an artificial heart valve according to the present invention.
Fig. 4-3 shows a schematic cross-sectional view of yet another delivery system for an artificial heart valve according to the present invention.
Fig. 4-4 shows a schematic cross-sectional view of a further delivery system for an artificial heart valve according to the present invention.
Fig. 5-1 schematically illustrates an artificial heart valve implantation process using the delivery system of Fig. 4-1.
Fig. 5-2 schematically illustrates an artificial heart valve implantation process using the delivery system of Fig. 4-2 or 4-3.
Fig. 5-3 schematically illustrates an artificial heart valve implantation process using the delivery system of Fig. 4-2 or 4-4.

### List of Reference Numerals

1 Outer Stent
11 Outer Stent Frame
111 Flange
111a First Flange Section
111b Second Flange Section
111c Third Flange Section
112 Main Body
112aInner Wall
112bOuter Wall
113 Attachment
12 One-Time Leaflets
13 Anchor
2 Inner Stent
21 Inner Stent Frame
211 Inflow Section
212 Outflow Section
22 Prosthetic Leaflets
23 Inner Stent Skirt
31 Delivery Sheath
32 Guidewire Lumen
33 Stent Catheter
331 Outer Stent Tube
332 Inner Stent Tube
33a Broader Portion
34 Bending-Control Sheath

### DETAILED DESCRIPTION

Embodiments of the present invention will be described below by means of specific examples. From the disclosure herein, other advantages and benefits of the invention can be readily appreciated by those familiar with the art.

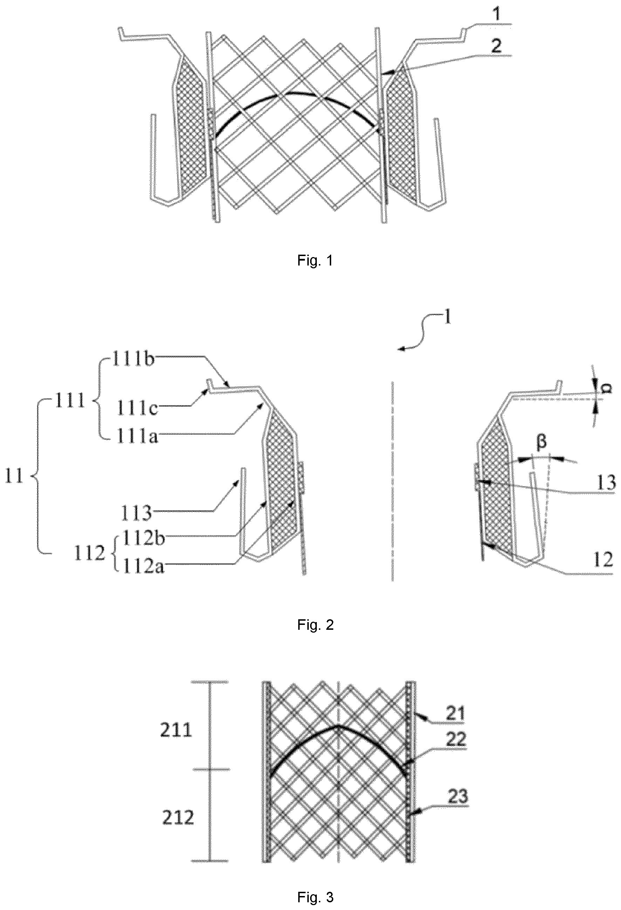

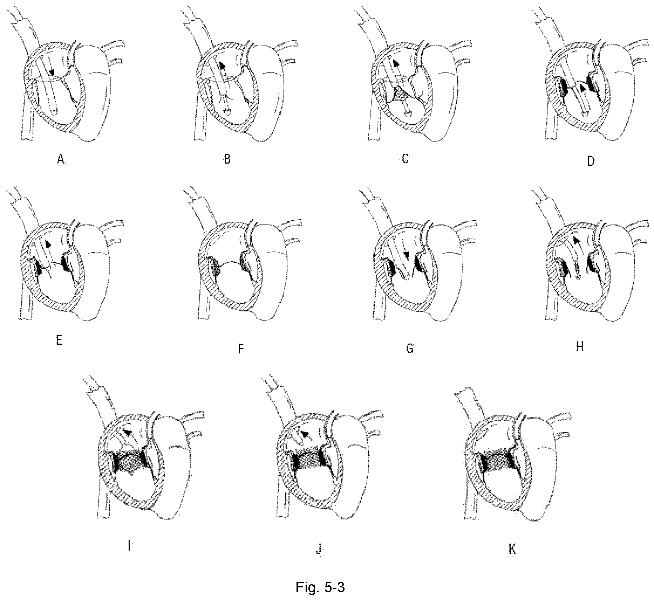

Reference is made to Figs. 1 to 5-3 below. It should be noted that architectural, proportional, dimensional and other details in the figures are presented only for the purpose of facilitating, in conjunction with the disclosure herein, the understanding and reading of those familiar with the art rather than being intended to limit conditions under which the present invention can be implemented. Therefore, they are technically of no substantive significance, and any and all architectural modifications, proportional variations or dimensional changes that do not affect the benefits and objects of the present invention are considered to fall within the scope of the teachings herein. Moreover, the terms "upper", "lower", "left", "right", "middle", "a", "an", "the" and the like are used herein merely for the sake of ease of explanation, rather than to limit the scope of implementation of the invention in any way. Any and all changes or modifications to relative relationships, as long as they do not alter the essence of the invention, are considered to fall within its scope of implementation.

As shown in Figs. 1 and 2, the present invention provides an artificial heart valve, the artificial heart valve includes an outer stent 1 and an inner stent 2. The outer stent 1 and the inner stent 2 may be detachably coupled to each other. Alternatively, during use of the artificial heart valve, the inner stent 2 may be disposed inside the outer stent 1 while the inner stent 2 may not be coupled to the stent 1. The outer stent 1 includes an outer stent frame 11 and one-time leaflets 12 attached to the outer stent frame 11. The inner stent 2 includes an inner stent frame 21 and prosthetic leaflets 22 attached to the inner stent frame 21.

In one embodiment, the inner stent frame 21 is tubular, and the outer stent 1 defines a cylindrical space allowing disposal of the inner stent frame 21 therein.

In one embodiment, the outer stent frame 11 includes, sequentially joined together, a flange 111, a main body 112 and an attachment 113.

After the artificial heart valve is implanted into a patient's body, the flange 111 is positioned in the atrium. Moreover, the flange 111 and the attachment 113 together clamp native tissue, thereby anchoring the valve.

In one embodiment, the flange 111 includes, sequentially joined together, a first flange section 111a, a second flange section 111b and a third flange section 111c.

In one embodiment, when the outer stent frame 11 is oriented upright, the second flange section 111b is inclined at an angle α ranging from -10° to 55° with respect to a horizontal direction. That is, in the outer stent frame 11, the second flange section 111b is inclined at an angle of -10° to 55° with respect to a radial direction of the outer stent frame 11.

In one embodiment, the main body 112 includes inner wall 112a and an outer wall 112b, the inner wall 112a and the outer wall 112b are joined together at both ends and thereby define an enclosure. Specifically, the inner wall 112a is closer to a center axis of the artificial heart valve, while the outer wall 112b is farther away from the center axis of the artificial heart valve. The inner wall 112a is used to support the inner stent 2, and the outer wall 112b enables the outer stent 1 to compress the native annulus and thereby create a radial anchoring force between the atrium and the ventricle.

The enclosure defined by the main body 112 is hollow.

In one embodiment, the attachment 113 includes, sequentially joined together, a first attachment section 113a, a second attachment section 113b and a third attachment section 113c.

In one embodiment, when the outer stent frame 11 is oriented upright, the third attachment section 113c is inclined at an angle β ranging from -15° to 15° with respect to a vertical direction. That is, the third attachment section 113c is inclined at an angle of -15° to 15° with respect to an axial direction of the outer stent frame 11.

Other angles of the attachment 113 may be arbitrary, as long as it is ensured that the angle between the third attachment section 113c and the vertical direction is within the aforementioned range. The attachment 113 may define a U-shaped space together with the outer wall 112b.

The attachment 113 can hook and catch the leaflets and pass between the chordae tendineae, preventing circumferential rotation of the outer stent 1.

The flange 111, the main body 112 and the attachment 113 cooperate to anchor the outer stent 1 between the atrium and the ventricle.

In one embodiment, the one-time leaflets 12 are provided on the inner wall 112a.

In one embodiment, the one-time leaflets 12 are braided and made of a polymer. The one-time leaflets 12 mainly function to serve as temporary prosthetic leaflets for preventing regurgitation over a period of time from release of the outer stent 1 to release of the inner stent 2. After the inner stent 2 is released, the one-time leaflets 12 are packed between the inner and outer stents to increase friction between, resulting in a greater anchoring force. Moreover, packing the one-time leaflets 12 between the inner and outer stents can reduce leakage therebetween and facilitate endothelialization. If the one-time leaflets 12 are absent, significant regurgitation that tends to cause heart failure may occur over the period from release of the outer stent 1 to release of the inner stent 2 during implantation of the artificial heart valve.

In one embodiment, the outer stent 1 further includes an outer stent skirt, the outer stent skirt is provided at least on surfaces of the flange 111 and the main body 112.

The outer stent skirt covers the surfaces of the flange 111 and the main body 112. The outer stent skirt may be only piece spanning both the surfaces of the flange 111 and the main body 112. Alternatively, the outer stent skirt may consist of two or more pieces spanning the respective surfaces of the flange 111 and the main body 112.

The outer stent skirt serves to, for example, block atrioventricular circulation and facilitate endothelialization after the outer stent 1 is implanted.

In one embodiment, the outer stent 1 further includes an anchor 13, the anchor 13 is disposed on the outer stent frame 11. In one preferred embodiment, the anchor 13 is provided on the inner wall 112a of the outer stent frame 11. The present invention is not limited to any particular location of the inner wall 112a where the anchor 13 is provided. In one preferred embodiment, the anchor 13 is provided at a central location of the inner wall 112a.

The anchor 13 may be a protrusion, which may be integrally formed with the inner wall 112a, or fixedly attached to the inner wall 112a as a separate component. Without limiting the scope of the present invention, the anchor 13 may be made of a biocompatible metallic or synthetic material. In one preferred embodiment, the anchor 13 has a rough surface, which can facilitate release of the inner stent 2 by providing an anchoring place and force.

The one-time leaflets 12 are provided below the anchor 13. This arrangement can ensure that an empty cavity is less likely to be left after the implantation of the inner stent 2, reducing the risk of thrombosis. Further, it can facilitate suturing and help avoid leakage from the ventricle to the atrium.

The inner stent frame 21 includes an inflow section 211 and an outflow section 212 joined to the inflow section 211. The inflow section 211 is adapted for passage of blood therethrough from the atrium into the inner stent 2, and the outflow section 212 is adapted for passage of blood therethrough from the inner stent 2 into the ventricle.

The inner stent frame 21 is made of a material selected from nickel-titanium alloy and a cobalt-chromium alloy. In the former case, the inner stent frame 21 is self-expanding, while in the latter case, it requires an expanding balloon.

The present invention is not limited to any particular material of the prosthetic leaflets 22, and they may be made of any suitable material commonly or generally used in the prior art. In one embodiment, the prosthetic leaflets 22 are made of biological tissue such as chemically stabilized tissue originated from a heart valve of an animal (e.g., pig), or of pericardial tissue harvested from an animal such as a cow (bovine pericardia), a sheep (ovine pericardia), a pig (porcine pericardia) or a horse (equine pericardia), with bovine pericardial tissues being preferred. In another embodiment, they may be made of small intestinal submucosal tissue. In other embodiments, they may be made of a synthetic material, such as expanded polytetrafluoroethylene (PTFE) or polyester. Optionally, examples of the synthetic material may further include thermoplastic polycarbonate-urethane, polyether-urethane, segmented polyether-urethane, silicone-polyether-urethane, silicone-polycarbonate-urethane and ultra-high molecular weight polyethylene. Examples of the synthetic material may further include biocompatible polymers such as polyolefins, elastomers, polyethylene glycol, polyethersulfone, polysulfone, polyvinylpyrrolidone, polyvinyl chloride, other fluoropolymers, silicone polyesters, silicone polymers and/or oligomers and/or polylactones, and block copolymers thereof.

In one embodiment, an anticoagulant may be applied to surfaces of the prosthetic leaflets 22. Examples of the anticoagulant may include, but are not limited to, heparinized polymers.

In one embodiment, the inner stent 2 further includes an inner stent skirt 23. The present invention is not limited to any particular location of the inner stent skirt 23, as long as the skirt is allowed to block atrioventricular circulation. In one embodiment, the inner stent skirt 23 is provided on an inner surface of the inner stent frame 21.

In one embodiment, the prosthetic leaflets 22 are attached to the inner stent skirt 23. The inner stent skirt 23 provides a place for attachment of the prosthetic leaflets 22 thereto.

The present invention is not limited to any particular materials of the outer and inner stent skirts 23, and each of them may be made of any suitable material commonly or generally used in the prior art. For example, each of them may be knitted, woven or braided and made of polyester, PTFE, expanded PTFE (ePTFE), etc. The outer and inner stent skirts 23 function mainly as seals for preventing regurgitation or perivalvular leakage.

The present invention is not limited to any particular materials of the outer stent 1 and the inner stent 2, and each of them may be made of any suitable material commonly or generally used in the prior art, such as nitinol, a titanium alloy, a cobalt-chromium alloy, MP35n, 316 stainless steel, L605, Phynox/Elgiloy, a platinum-chromium alloy, or another biocompatible metal known to those skilled in the art, or implemented as a laser-cut solid metal tube. Preferably, each of them is made of a shape memory alloy. For example, each of them may be made of an elastically or plastically deformable material, such as a balloon expandable material, or a shape memory alloy which can transition in response to temperature changes between a collapsed configuration for delivery and an expanded or unfolded configuration. Optionally, each stent may be made of braid wires or another suitable material.

As the outer stent 1 and inner stent 2 are detachably coupled, or not coupled, to each other, the outer stent 1 may be first implanted into a patient's body and firmly anchored between the atrium and the ventricle, followed by release of the inner stent 2. The inner stent 2 may have a mesh-like structure, which may self-expand or be expanded by a balloon, and thereby be seated within the outer stent. This design with the separable inner and outer stents allows a delivery sheath in the delivery system to have a reduced radial dimension (e.g., from 35 Fr or greater to 28 Fr for a first delivery system, and from 35 Fr or greater to 24 Fr for a second or third delivery system), thereby solving the problem of delivery difficulties resulting from excessive size associated with existing delivery systems for double-layer stents. Thus, higher implantation safety and dimensions suitable for a wider range of applications can be achieved.

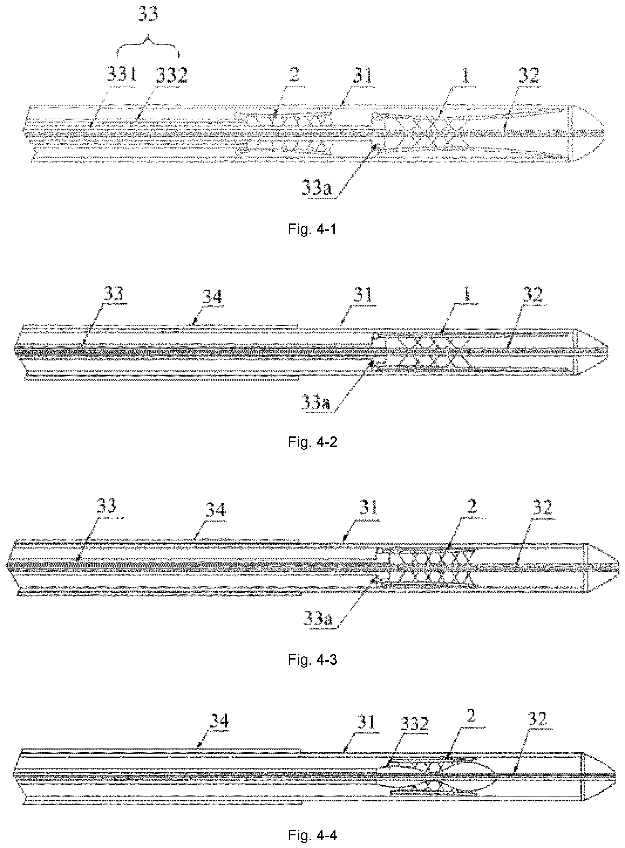

As shown in Figs. 4-1 to 4-4, the present invention also provides a delivery system for the artificial heart valve. The delivery system includes a delivery sheath 31, as well as disposed within the delivery sheath 31, a guidewire lumen 32 and a stent catheter 33. The stent catheter 33 is adapted to engage the artificial heart valve.

In the implementation shown in Fig. 4-1 (first delivery system), the stent catheter 33 includes an outer stent tube 331 and an inner stent tube 332, the outer stent tube 331 and the inner stent tube 332 are arranged in such a manner that one protrudes out of the other and are both tubular in shape. In this implementation, the inner stent frame 21 is made of a nickel-titanium alloy, the inner stent frame 21 can self-expand.

The inner stent tube 332 has a greater cross-sectional area than the outer stent tube 331. The outer stent tube 331 is inserted in the inner stent tube 332. The inner stent tube 332 has a constant diameter. One end of the inner stent tube 332 is engaged with the inner stent 2. The outer stent tube 331 has a broader portion 33a at its one end, the broader portion 33a is adapted for engagement with the outer stent 1.

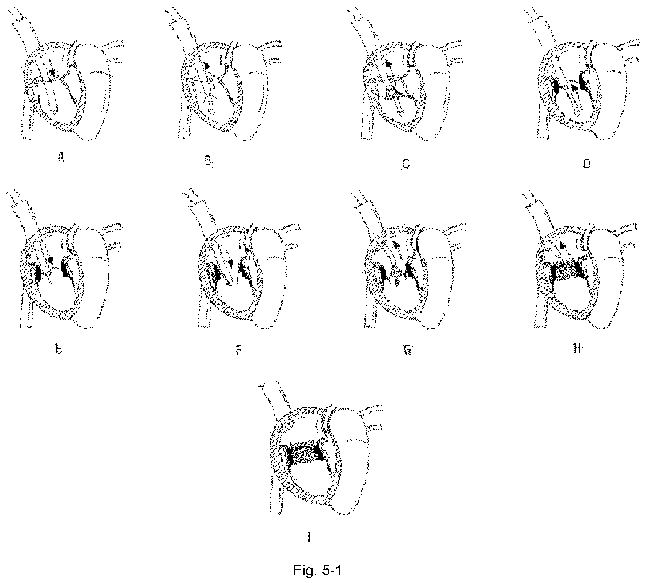

As shown in Fig. 5-1, an artificial heart valve implantation process using the delivery system may involve the steps as follows. At first, the inner and outer stents are crimped on the inner stent tube 332 and the outer stent tube 331, respectively, and then implanted. During the implantation, the delivery sheath 31 is retracted to release the outer stent 1. After the outer stent 1 is released, the guidewire lumen 32 is retracted to a location near the inner stent 2. The delivery system is adjusted so that the inner stent 2 becomes coaxial with the outer stent 1 (the coaxiality can be achieved with the aid of the delivery system). The delivery sheath 31 is further retracted to release the inner stent 2. After the release is completed, the delivery sheath 31 and the delivery system are withdrawn.

In the implementation shown in Figs. 4-2 and 4-3 (second delivery system), the delivery system further includes a bending-control sheath 34 fitted over the delivery sheath 31. The stent catheter 33 is tubular in shape. The stent catheter 33 has a broader portion 33a at one end, the broader portion 33a is adapted to engage the outer stent 1 or the inner stent 2.

In this implementation, the inner stent frame 21 is made of a nickel-titanium alloy, and the inner stent frame 21 can self-expand.

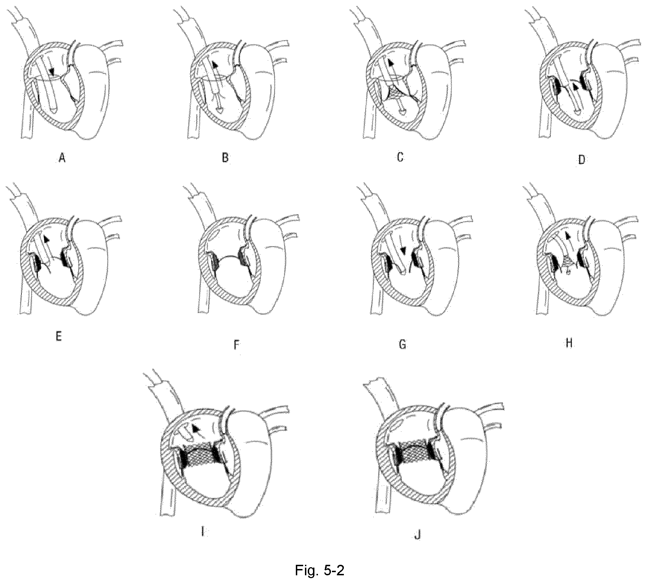

As shown in Fig. 5-2, an artificial heart valve implantation process can be performed using two such delivery systems and may involve the steps as follows. At first, the inner and outer stents are respectively loaded in the systems. The outer stent 1 is first implanted. In this process, the corresponding delivery system is passed through the bending-control sheath 34, and the outer stent 1 is released between the atrium and the ventricle by retracting the delivery sheath 31. After that, this delivery system is withdrawn. The other delivery system in which the inner stent 2 is loaded is then passed through the bending-control sheath 34, and the inner stent 2 is released, also by retracting the delivery sheath 31, into the cylindrical space of the outer stent 1 located between the atrium and the ventricle.

In the implementation shown in Figs. 4-4 (third delivery system), the delivery system further includes a bending-control sheath 34 fitted over the delivery sheath 31. The stent catheter 33 is in the shape of a balloon. The stent catheter 33 is adapted to be accommodated in the inner stent 2.

In this implementation, the inner stent frame 21 is made of a cobalt-chromium alloy, and the inner stent frame 21 can be expanded by the balloon.

As shown in Fig. 5-3, an artificial heart valve implantation process can be performed using the delivery system shown in Fig. 4-2 and the delivery system shown in Fig. 4-4 and may involve the steps as follows. At first, the outer stent 1 is loaded in the delivery system of Fig. 4-2 and the inner stent 2 in the delivery system of Fig. 4-4. The outer stent 1 is first implanted. Subsequently, the delivery system in which the inner stent 2 is loaded is passed through the bending-control sheath 34. When reaching a location between the atrium and the ventricle, the delivery sheath 31 first retracted, and a physiological saline solution is filled into the stent catheter 33 to expand the inner stent 2, thereby seating the inner stent 2 in the inner wall 112a of the outer stent 1 centrally about the center thereof.

The present invention also provides a heart valve replacement system including the artificial heart valve and the delivery system as defined above.

In summary, the present invention has effectively overcome the various drawbacks of the prior art and has a high value in industrial use.

The embodiments disclosed hereinabove are solely for the purpose of exemplary illustration of the principles and benefits of the present invention and not for the purpose of limiting the invention. Any person familiar with the art can make modifications or changes to the disclosed embodiments without departing from the spirit and scope of this invention. Accordingly, any and all equivalent modifications or changes made by any person with general common knowledge in the art without departing from the spirit and teachings of the present application are intended to be embraced within the scope as defined by the appended claims.

## Claims

1. An artificial heart valve, comprising an outer stent (1) and an inner stent (2), the outer stent (1) detachably connected to the inner stent (2), or the inner stent (2) located inside the outer stent (1) during use of the artificial heart valve, the outer stent (1) comprising an outer stent frame (11) and one-time leaflets (12) provided on the outer stent frame (11), the inner stent (2) comprising an inner stent frame (21) and prosthetic leaflets (22) disposed within the inner stent frame (21).

2. The artificial heart valve according to claim 1, wherein the outer stent frame (11) comprises a flange (111), a main body (112) and an attachment (113) connected in sequence, the flange (111) comprising a first flange section (111a), a second flange section (111b) and a third flange section (111c) connected in sequence, when the outer stent frame (11) is oriented upright, the second flange section (111b) is inclined at an angle α of -10° to 55° with respect to a horizontal direction, the main body (112) comprising an inner wall (112a) and an outer wall (112b), the inner wall (112a) and the outer wall (112b) are connected together at both ends to form an enclosure, the attachment (113) comprising a first attachment section (113a), a second attachment section (113b) and a third attachment section (113c) connected in sequence, when the outer stent frame (11) is oriented upright, the third attachment section (113c) is inclined at an angle β of -15° to 15° with respect to a vertical direction.

3. The artificial heart valve according to claim 2, wherein the one-time leaflets (12) are provided on the inner wall (112a).

4. The artificial heart valve according to claim 2, wherein the outer stent (1) further comprises an outer stent skirt, and the outer stent skirt is provided at least on surfaces of the flange (111) and the main body (112).

5. The artificial heart valve according to claim 1, wherein the outer stent (1) further comprises an anchor (13), and the anchor (13) is provided on the outer stent frame (11).

6. The artificial heart valve according to claim 2, wherein the anchor (13) is provided on the inner wall (112a).

7. The artificial heart valve according to claim 1, wherein the inner stent frame (21) is divided into an inflow section (211) and an outflow section (212), wherein the inner stent (2) further comprises an inner stent skirt (23), and wherein the inner stent skirt (23) is provided on an inner surface of the inner stent frame (21).

8. The artificial heart valve according to claim 7, wherein the prosthetic leaflets (22) are attached to the inner stent skirt (23).

9. A delivery system for an artificial heart valve, comprising a delivery sheath (31) and, disposed within the delivery sheath (31), a guidewire lumen (32) and a stent catheter (33), the stent catheter (33) configured to be suitable for engagement with the artificial heart valve according to any one of claims 1 to 8.

10. The delivery system according to claim 9, wherein the stent catheter (33) comprises an outer stent tube (331) and an inner stent tube (332), the outer stent tube (331) inserted in the inner stent tube (332) and the outer stent tube (331) partially extending out of the inner stent tube (332), one end of the inner stent tube (332) configured for engagement with the inner stent (2), the outer stent tube (331) comprising a broader portion (33a) at one end thereof, the broader portion (33a) configured for engagement with the outer stent (1), the outer stent tube (331) and the inner stent tube (332) both tubular in shape.

11. The delivery system according to claim 9, further comprising a bending-control sheath (34) fitted over the delivery sheath (31), wherein the stent catheter (33) is tubular in shape and one end of the stent catheter (33) is provided with a broader portion (33a), the broader portion (33a) is configured for engagement with the outer stent (1) or the inner stent (2).

12. The delivery system according to claim 9, further comprising a bending-control sheath (34) fitted over the delivery sheath (31), wherein the stent catheter (33) is in the shape of a balloon, and the stent catheter (33) is configured to be received in the inner stent (2) during implantation.

13. A heart valve replacement system, comprising the artificial heart valve according to any one of claims 1 to 8 and the delivery system according to any one of claims 9 to 12.
